# EUROPEAN PATENT APPLICATION

(11) **EP 3 075 733 A1**
(43) Date of publication of application: **05.10.2016**
(21) Application number: 15305473.9
(22) Date of filing: 31.03.2015
(51) Int. Cl.: C07D 471/08, A61K 31/439, A61P 31/04

(54) **NOVEL HETEROCYCLIC COMPOUNDS AND THEIR USE IN PREVENTING OR TREATING BACTERIAL INFECTIONS**

(71) Applicant: Mutabilis, 75007 Paris (FR)
(72) Inventor: Caravano, Audrey, 95880 Enghien les Bains (FR); Chasset, Sophie, 77176 Nandy (FR); Chevreuil, Francis, 60500 Chantilly (FR); Faivre, Fabien, 93700 Drancy (FR); Lecointe, Nicolas, 75018 Paris (FR); Ledoussal, Benoît, 22450 Pommerit Jaudy (FR); Le Strat, Frédéric, 77380 Combs la Ville (FR); Richard, Sébastien, 75020 Paris (FR); Simon, Christophe, 94550 Chevilly Larue (FR); Vomscheid, Sophie, 75014 Paris (FR)
(74) Representative: Lavoix

(57) **Abstract**

The present invention relates to heterocyclic compounds, their process of preparation, pharmaceutical compositions comprising these compounds and use thereof, optionally in combination with other antibacterial agents and/or beta-lactam compounds, for the prevention or treatment of bacterial infections. The present invention also relates to the use of these compounds as β-lactamase inhibitors and/or as antibacterial agents.

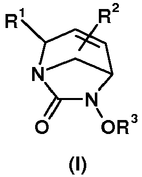

## Description

The present invention relates to heterocyclic compounds, their process of preparation, the pharmaceutical compositions comprising these compounds and use thereof, optionally in combination with other antibacterial agents and/or beta-lactams, for the prevention or treatment of bacterial infections. The present invention also relates to the use of these compounds as beta-lactamase inhibitors and/or antibacterial agent.

It has been described that there is a continuous evolution of antibacterial resistance which could lead to bacterial strains against which known antibacterial compounds are inefficient.

There is thus a need to provide novel compounds and composition that can overcome bacterial antibiotic resistance.

The objective of the present invention is to provide new heterocyclic compounds that can be used as antibacterial agent and/or beta-lactamase inhibitor.

An objective of the present invention is also to provide new heterocyclic compounds that can be used for the prevention or treatment of bacterial infections.

Another objective of the present invention is to provide such new compounds which can overcome bacterial antibiotic resistance.

An objective of the invention is also to provide composition comprising these new heterocyclic compounds, optionally in combination with one or more other antibacterial agent, for the prevention or treatment of bacterial infections and which can overcome bacterial antibiotic resistance.

Other objectives will appear throughout the following description of the invention.

The present invention relates to compound of formula (I) 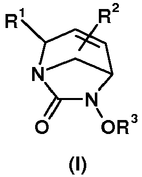 wherein
R¹ represents a carbon-linked 4-, 5- or 6-membered heterocycle saturated, partially or totally unsaturated or aromatic comprising at least one nitrogen atom, -CN, -(CH₂)ₘ-OQ¹, -(CH₂)m-OC(O)Q¹, -C(O)OQ¹, -(CH₂)ₘ-OC(O)OQ¹, -(CH₂)ₘ-OC(O)NQ¹Q², -C(O)NHQ¹, -C(O)NHOQ¹, -(CH₂)ₘ-NHS(O)₂NQ¹Q², -C(O)NH-NHQ¹, -C(O)O-NHQ¹, -(CH₂)ₘ-NHC(O)Q¹,-(CH₂)ₘ-NHS(O)₂Q¹, -(CH₂)ₘ-NHC(O)OQ¹, -(CH₂)ₘ-NHC(O)NQ¹Q², -(CH₂)ₘ-NHQ³, -(CH₂)ₘ-NH-C(NHQ³)=NQ⁴, (CH₂)ₘ-NH-CH=NQ³, -C(NHQ³)=NQ⁴, wherein the heterocycle is optionally substituted by one or more T¹;
R² represents a 4- to 10-membered heterocycle saturated, partially or totally unsaturated or aromatic optionally substituted by one or more T²;
R³ represents -SO₃H, -CFHCOOH or -CF₂COOH;
T¹, identical or different independently represents a fluorine atom, a (C₁-C₃)-alkyl, a (C₁-C₃)-fluoroalkyl, O-(C₁-C₃)fluoroalkyl, -(CH₂)ₙ-heterocycle wherein the heterocycle is a 4-, 5- or 6-membered heterocycle saturated, partially or totally unsaturated or aromatic comprising at least one nitrogen atom, -(CH₂)ₙOQ¹, -(CH₂)ₙ-C(O)ONHQ¹, -(CH₂)ₙ-CN, -(CH₂),-OC(O)Q¹, -(CH₂)ₙ-C(O)OQ¹, -(CH₂)ₙ-NHS(O)₂NQ¹Q², -(CH₂)ₙ-OC(O)OQ¹, -(CH₂)ₙ-OC(O)NHQ¹, -(CH₂)ₙ-C(O)NHQ¹, -(CH₂)ₙ-C(O)NHOQ¹, -(CH₂)ₙ-C(O)NH-NHQ¹, -(CH₂)ₙ-NHC(O)Q¹, -(CH₂)ₙ-NHS(O)₂Q¹, -(CH₂)ₙ-NHC(O)OQ¹, -(CH₂)ₙ-NHC(O)NQ¹Q^{2,} -(CH₂)ₙ-NHQ¹, -(CH₂)ₙ-NH-C(NHQ³)=NQ⁴, -(CH₂)ₙ-NH-CH=NQ³, (CH₂)ₙ-C(NHQ³)=NQ⁴, wherein the alkyl, fluoroalkyl, O-fluoroalkyl, and -(CH₂)ₙ-heterocycle are independently optionally substituted by one or more T³;
Q¹ and Q², identical or different independently represent a hydrogen atom, (C₁-C₃)alkyl, -(CH₂)_{q}-NHQ³, -(CH₂)_{q}-NH-C(NHQ³)=NQ⁴, (CH₂)_{q}-NH-CH=NQ³, (CH₂)ᵣ-C(NHQ³)=NQ⁴, -(CH₂)_{q}-OQ³, -(CH₂)ᵣ-CONHQ³, -(CH₂)ₙ-heterocycle, wherein the heterocycle is a 4-, 5- or 6-membered heterocycle saturated, partially or totally unsaturated or aromatic comprising at least one nitrogen atom; wherein the alkyl and -(CH₂)ₙ-heterocycle are independently optionally substituted by one or more T³; or
   Q¹ and Q² and the nitrogen atom to which they are bonded form together a saturated or partially unsaturated 4-, 5- or 6-membered heterocycle optionally substituted by one or more **T**³;
Q³ and Q⁴, identical or different, independently represent a hydrogen atom or (C₁-C₃)alkyl;
T² , identical or different, independently represents a fluorine atom, (C₁-C₃)alkyl, (C₁-C₃)fluoroalkyl, O-(C₁-C₃)fluoroalkyl, (X)ₚ-(CH₂)ₙ-(C₃-C₆)cycloalkyl, (X)ₚ-(CH₂)ₙ-(C₃-C₆)cyclofluoroalkyl, -(X)ₚ-(CH₂)ₙ-heterocycle wherein the heterocycle is a 4-, 5- or 6-membered heterocycle, saturated, partially or totally unsaturated or aromatic, -(X)ₚ(CH₂)ₜOQ⁵, (X)ₚ-(CH₂)ᵤ-CN, -(X)ₚ-(CH₂)ₜ-OC(O)Q⁵, (X)ₚ-(CH₂)ᵤ-C(O)OQ⁵, (X)ₚ-(CH₂)ₜ-C(O)OQ⁵, (X)ₚ-(CH₂)ₜ-OC(O)NQ⁵Q⁶, (X)ₚ-(CH₂)ᵤ-C(O)NQ⁵Q⁶, (X)ₚ-(CH₂)ᵤ-C(O)ONQ⁵Q⁶ , (X)ₚ-(CH₂)ᵤ-C(O)NQ⁵OQ⁶, (X)ₚ-(CH₂)ᵤ-C(O)NQ⁵-NQ⁵Q⁶, (X)ₚ-(CH₂)ₜ-NQ⁵C(O)Q⁶, (X)ₚ-(CH₂)ₜ-NQ⁵S(O)₂NQ⁵Q⁶, (X)ₚ-(CH₂)ₜ-NQ⁵S(O)₂Q⁶, (X)ₚ-(CH₂)ₜ-NQ⁵C(O)OQ⁶, (X)ₚ-(CH₂)ₜ-NQ⁵C(O)NQ⁵Q⁶, (X)ₚ-(CH₂)ₜ-NQ⁵Q⁶, (X)ₚ-(CH₂)ₜ-NH-C(NHQ³)=NQ⁴, (X)ₚ-(CH₂)ₜ-NH-CH=NQ³, (X)ₚ-(CH₂)ᵤ-C(NHQ³)=NQ⁴, wherein the alkyl, fluoroalkyl, O-fluoroalkyl, (X)ₚ-(CH₂)ₙ-cycloalkyl, (X)ₚ-(CH₂)ₙ-cyclofluoroalkyl, -(X)ₚ-(CH₂)ₙ-heterocycle are independently optionally substituted by one or more T³;
Q⁵ and Q⁶, identical or different independently represent a hydrogen atom, (C₁-C₃)alkyl, -(CH₂)_{q}-NHQ³, -(CH₂)_{q}-NH-C(NHQ³)=NQ⁴, (CH₂)_{q}-NH-CH=NQ³, (CH₂)ᵣ-C(NHQ³)=NQ⁴, -(CH₂)_{q}-OQ³, -(CH₂)ᵣ-CONHQ³ -(CH₂)ₙ-heterocycle, wherein the heterocycle is a 4-, 5- or 6-membered heterocycle saturated, partially or totally unsaturated or aromatic comprising at least one nitrogen atom; wherein the alkyl and -(CH₂)ₙ-heterocycle are independently optionally substituted by one or more T³; or
   Q⁵ and Q⁶ and the nitrogen atom to which they are bonded form together a saturated or partially unsaturated 4-, 5- or 6-membered heterocycle optionally substituted by one or more T³;
T³, identical or different, independently represents -OH, -NH₂, -CONH₂;
m, identical or different, independently represents 1 or 2;
   n, identical or different, independently represents 0,1, 2 or 3;
   t, identical or different, independently represents 0, 1, 2 or 3;
   u, identical or different, independently represents 0, 1, 2 or 3;
   q, identical or different, independently represents 2 or 3;
   r, identical or different, independently represents 1, 2 or 3;
   p, identical or different, independently represents 0 or 1 and when p is 0 t, identical or different is 0, 1, 2 or 3 and u, identical or different, is 0, 1, 2 or 3 and when p is 1 t, identical or different, independently represents 2 or 3 and u, identical or different, represents 1, 2 or 3; X, identical or different, independently represents O, S, S(O), S(O)₂ or N(Q³);
   wherein
   - any carbon atom present within a group selected from alkyl ; cycloalkyl ; fluoroalkyl ; cyclofluoroalkyl ; heterocycle can be oxidized to form a C(O) group;
   - any sulphur atom present within an heterocycle can be oxidized to form a S(O) group or a S(O)₂ group ;
   - any nitrogen atom present within a group wherein it is trisubstituted (thus forming a tertiary amine) or within an heterocycle can be further quaternized by a methyl group;
   or their pharmaceutically acceptable salts, their corresponding zwitterion, or their optical isomers, racemates, diastereoisomers, enantiomers or tautomers.

Preferably, the compounds of formula (I) are chosen among compounds of formula (I*) 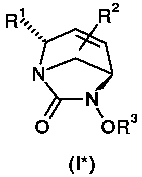 wherein R¹, R² and R³ are as defined for compounds of formula (I).

Preferably, the compounds of formula (I) are chosen among compounds of formula (A) or (B) 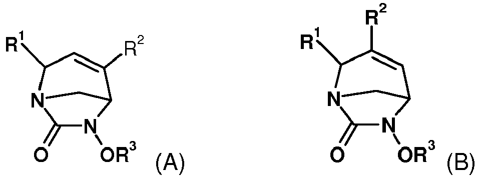 wherein R¹, R² and R³ are as defined for compounds of formula (I).

Preferably, the compounds of formula (I) are chosen among compounds of formula (A*) or (B*) 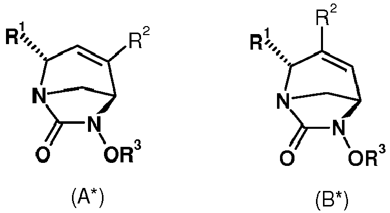 wherein R¹, R² and R³ are as defined for compounds of formula (I).

In one embodiment of the present invention, in the compounds of formula (I), (I*), (A), (A*), (B) and (B*), R¹ preferably represents a carbon-linked 4-, 5- or 6-membered heterocycle saturated, partially or totally unsaturated or aromatic comprising at least one nitrogen atom, -CN, -(CH₂)ₘ-OQ¹, -(CH₂)ₘ-OC(O)Q¹, -C(O)OQ¹, -(CH₂)ₘ-OC(O)OQ¹, -(CH₂)ₘ-OC(O)NQ¹Q², -C(O)NHQ¹, -(CH₂)ₘ-NHS(O)₂NQ₁Q₂, -C(O)NHOQ¹, -C(O)NH-NHQ¹, -C(O)O-NHQ¹,-(CH₂)ₘ-NHC(O)Q¹, -(CH₂)ₘ-NHS(O)₂Q¹, -(CH₂)ₘ-NHC(O)OQ¹, -(CH₂)ₘ-NHC(O)NQ¹Q², wherein the heterocycle is optionally substituted by one or more T¹, wherein m, Q¹, Q² and T¹ are as defined above. Preferably R¹ represents a carbon-linked 4-, 5- or 6-membered heterocycle saturated, partially or totally unsaturated or aromatic comprising at least one nitrogen atom, CN, -C(O)NHQ¹, -C(O)NHOQ¹, -C(O)NH-NHQ¹ or -(CH₂)ₘOQ¹ wherein the heterocycle is optionally substituted by one or more T¹, wherein m, Q¹ and T¹ are as defined above.

In another embodiment of the present invention, in the compounds of formula (I), (I*), (A), (A*), (B) and (B*), R¹ preferably represents -(CH₂)ₘNHQ³, -(CH₂)ₘ-NH-C(NHQ³)=NQ⁴, (CH₂)ₘ-NH-CH=NQ³, -C(NHQ³)=NQ⁴, wherein m, Q³ and Q⁴ are as defined above. Preferably R¹ represents -(CH₂)ₘNHQ³, -(CH₂)ₘ-NH-C(NHQ³)=NQ⁴, wherein m, Q³ and Q⁴ are as defined above.

According to another embodiment of the present invention, in the compounds of formula (I), (I*), (A), (A*), (B) and (B*), R¹ preferably represents a carbon-linked 4-, 5- or 6-membered heterocycle saturated, partially or totally unsaturated or aromatic comprising at least one nitrogen atom, -(CH₂)ₘOC(O)Q¹, -C(O)OQ¹, -(CH₂)ₘ-NHS(O)₂NQ¹Q², -(CH₂)ₘ-OC(O)OQ¹,-C(O)NHOQ¹, -C(O)NH-NHQ¹ ,-C(O)O-NHQ¹, -(CH₂)ₘ-NHC(O)Q¹, -(CH₂)ₘNHS(O)₂Q¹,-(CH₂)ₘ-NHC(O)OQ¹, -(CH₂)ₘ-NHC(O)NQ¹Q², -(CH₂)ₘ-NH-C(NHQ³)=NQ⁴, (CH₂)ₘ-NH-CH=NQ³ or -C(NHQ³)=NQ⁴, wherein the heterocycle is optionally substituted by one or more T¹, wherein m, Q¹, Q², Q³, Q⁴ and T¹ are as defined above.

According to one embodiment of the present invention, in the compounds of formula (I), (I*), (A), (A*), (B) and (B*), R¹ preferably represents -(CH₂)ₘ-NH-C(NHQ³)=NQ⁴, (CH₂)ₘ-NH-CH=NQ³, -C(NHQ³)=NQ⁴, wherein m, Q³ and Q⁴ are as defined above. Preferably R¹ represents -C(NHQ³)=NQ⁴, wherein m, Q³ and Q⁴ are as defined above.

According to a further embodiment of the present invention, in the compounds of formula (I), (I*), (A), (A*), (B) and (B*), R¹ preferably represents a carbon-linked 4-, 5- or 6-membered heterocycle saturated, partially or totally unsaturated or aromatic comprising at least one nitrogen atom, -(CH₂)ₘOC(O)Q¹, -C(O)OQ¹, -(CH₂)ₘ-OC(O)OQ¹, -(CH₂)ₘ-NHS(O)2NQ¹Q²,-C(O)NHOQ¹, -C(O)NH-NHQ¹, -C(O)O-NHQ¹, -(CH₂)ₘ-NHC(O)Q¹, -(CH₂)ₘNHS(O)₂Q¹,-(CH₂)ₘ-NHC(O)OQ¹, -(CH₂)ₘ-NHC(O)NQ¹Q² wherein the heterocycle is optionally substituted by one or more T¹ wherein m, Q¹, Q² and T¹ are as defined above. Preferably, R¹ represents a carbon-linked 4-, 5- or 6-membered heterocycle saturated, partially or totally unsaturated or aromatic comprising at least one nitrogen atom, -C(O)NHOQ¹ or -C(O)NH-NHQ¹, wherein the heterocycle is optionally substituted by one or more T¹, wherein m, Q¹ and T¹ are as defined above.

In the compounds of formula (I), (I*), (A), (A*), (B) and (B*) according to the invention, when R¹ is a carbon-linked 4-, 5- or 6-membered heterocycle saturated, partially or totally unsaturated or aromatic comprising at least one nitrogen atom and optionally substituted by one or more T¹, it can comprise other heteroatoms, for example at least one further heteroatoms, for example 1, 2 or 3 further heteroatoms, the further heteroatom being preferably chosen among N, O, S, S(O) or S(O)₂. It is preferably a monocyclic heterocycle.

Preferably, in the compounds of formula (I), (I*), (A), (A*), (B) and (B*) according to the invention, R¹ represents a carbon-linked 4-, 5- or 6-membered heterocycle saturated, partially or totally unsaturated or aromatic comprising at least one nitrogen atom, optionally substituted by one or more T¹; -C(O)NHQ¹, -(CH₂)ₘ-NH-C(NHQ³)=NQ⁴ or -(CH₂)ₘNHQ³, wherein Q¹, Q³, Q⁴, T¹ and m are as defined above.

In the compounds of formula (I), (I*), (A), (A*), (B) and (B*) according to the invention, preferably R² is a monocyclic or bicyclic 4- to 10-membered heterocycle, saturated, partially or totally unsaturated or aromatic, optionally substituted by one or more T². Preferably, this heterocycle comprises at least one nitrogen atom and can comprise further heteroatoms, for example at least one further heteroatoms, for example 1, 2 or 3 further heteroatoms, the further heteroatom being preferably chosen among N, O, S, S(O) or S(O)₂.
Preferably, R² is a monocyclic 4-, 5- or 6-membered heterocycle, saturated, partially or totally unsaturated or aromatic, optionally substituted by one or more T². Preferably, this heterocycle comprises at least one nitrogen atom and can comprise further heteroatoms, for example at least one further heteroatoms, for example 1, 2 or 3 further heteroatoms, the further heteroatom being preferably chosen among N, O, S, S(O) or S(O)₂. Preferably, the heterocycle is a carbon-linked heterocycle.

Preferably, in the compounds of formula (I), (I*), (A), (A*), (B) and (B*) according to the invention, R² represents a 4-, 5- or 6-membered heterocycle, saturated, partially or totally unsaturated or aromatic, comprising at least one nitrogen atom and optionally another heteroatom chosen among O, S, S(O), S(O)₂ or N, optionally substituted by one or more T².

Preferably, in the compounds of formula (I), (I*), (A), (A*), (B) and (B*) according to the invention, R³ represent SO₃H or CF₂COOH.

Preferably, in the compounds of formula (I), (I*), (A), (A*), (B) and (B*), Q³ and Q⁴, identical or different independently represent H or methyl, preferably H.

Preferably, in the compounds of formula (I), (I*), (A), (A*), (B) and (B*), Q¹ and Q², identical or different independently represent H, methyl, -CH₂-CH₂-NH₂, -CH₂-CH₂-NH-CNH₂=NH, -CH₂-CH₂-NH-CH=NH, -CH₂-C(NH₂)=NH, CH₂-CH₂-OH, -CH₂-CONH₂, a -(CH₂)ₙ-heterocycle, wherein the heterocycle is a 4-, 5- or 6-membered heterocycle saturated, partially or totally unsaturated or aromatic comprising at least one nitrogen atom; wherein the heterocycle is independently optionally substituted by one or more T³, wherein n and T³ are as defined above.
Preferably, in the compounds of formula (I), (I*), (A), (A*), (B) and (B*), Q¹ and Q², identical or different independently represent H, methyl, -CH₂-CH₂-NH₂, -CH₂-CH₂-NH-CNH₂=NH, -CH₂-CH₂-NH-CH=NH, -CH₂-C(NH₂)=NH, CH₂-CH₂-OH, -CH₂-CONH₂, a saturated 4-membered heterocycle comprising one nitrogen atom, a saturated 5-membered heterocycle comprising one nitrogen atom, a saturated 6-membered heterocycle comprising one nitrogen atom; wherein the heterocycle is independently optionally substituted by one or more T³, wherein n and T³ are as defined above, preferably not substituted.

Preferably, in the compounds of formula (I), (I*), (A), (A*), (B) and (B*):
- R² is a monocyclic or bicyclic 4- to 10-membered heterocycle, saturated, partially or totally unsaturated or aromatic, optionally substituted by one or more T²; and
- R¹ represents a carbon-linked 4- to 6-membered heterocycle saturated, partially or totally unsaturated or aromatic comprising at least one nitrogen atom, -CN, -(CH₂)ₘ-OQ¹, -(CH₂)m-OC(O)Q¹, -C(O)OQ¹, -(CH₂)ₘ-OC(O)OQ¹, -(CH₂)ₘ-OC(O)NQ¹Q², - C(O)NHQ¹, -(CH₂)ₘ-NHS(O)₂NQ¹Q², -C(O)NHOQ¹, -C(O)NH-NHQ¹, -C(O)O-NHQ¹,-(CH₂)ₘ-NHC(O)Q¹, -(CH₂)ₘ-NHS(O)₂Q¹, -(CH₂)ₘ-NHC(O)OQ¹, -(CH₂)ₘ-NHC(O)NQ¹Q², wherein the heterocycle is optionally substituted by one or more T¹, wherein m, Q¹, Q² and T¹ are as defined above; or
- R¹ represents -(CH₂)ₘNHQ³, -(CH₂)ₘ-NH-C(NHQ³)=NQ⁴, (CH₂)ₘ-NH-CH₌NQ³,-C(NHQ³)=NQ⁴, wherein m, Q³ and Q⁴ are as defined above; or
- R¹ represents a carbon-linked 4-, 5- or 6-membered heterocycle saturated, partially or totally unsaturated or aromatic comprising at least one nitrogen atom, - (CH₂)ₘOC(O)Q¹, -C(O)OQ¹, -(CH₂)ₘ-NHS(O)₂NQ¹Q², -(CH₂)ₘ-OC(O)OQ¹, - C(O)NHOQ¹, -C(O)NH-NHQ¹ ,-C(O)O-NHQ¹, -(CH₂)ₘ-NHC(O)Q¹, -(CH₂)ₘNHS(O)₂Q¹, -(CH₂)ₘ-NHC(O)OQ¹, -(CH₂)ₘ-NHC(O)NQ¹Q², -(CH₂)ₘ-NH-C(NHQ³)=NQ⁴, (CH₂)ₘ-NH-CH=NQ³ or -C(NHQ³)=NQ⁴, wherein the heterocycle is optionally substituted by one or more T¹, wherein m, Q¹, Q², Q³, Q⁴ and T¹ are as defined above; or
- R¹ represents -(CH₂)ₘ-NH-C(NHQ³)=NQ⁴, (CH₂)ₘ-NH-CH=NQ³, (CH₂)-C(NHQ³)=NQ⁴, wherein m, Q³ and Q⁴ are as defined above ; or
- R¹ represents a carbon-linked 4- to 6-membered heterocycle saturated, partially or totally unsaturated or aromatic comprising at least one nitrogen atom, - (CH₂)ₘOC(O)Q¹, -C(O)OQ¹, -(CH₂)ₘ-OC(O)OQ¹, -(CH₂)m-NHS(O)₂NQ₁Q₂, - C(O)NHOQ¹, -C(O)NH-NHQ¹, -C(O)O-NHQ¹, -(CH₂)ₘ-NHC(O)Q¹, -(CH₂)ₘNHS(O)₂Q¹, -(CH₂)ₘ-NHC(O)OQ¹, -(CH₂)ₘ-NHC(O)NQ¹Q² wherein the heterocycle is optionally substituted by one or more T¹ wherein m, Q¹, Q² and T¹ are as defined above.

Preferably, in the compounds of formula (I), (I*), (A), (A*), (B) and (B*):
- R² is a monocyclic or bicyclic 4- to 10-membered heterocycle, saturated, partially or totally unsaturated or aromatic, optionally substituted by one or more T²; and
- R¹ represents a carbon-linked 4-, 5- or 6-membered heterocycle saturated, partially or totally unsaturated or aromatic comprising at least one nitrogen atom, -CN, -(CH₂)ₘ-OQ¹, -(CH₂)ₘ-OC(O)Q¹, -C(O)OQ¹, -(CH₂)ₘ-OC(O)OQ¹, -(CH₂)ₘ-OC(O)NQ¹Q², - C(O)NHQ¹, -(CH₂)ₘ-NHS(O)₂NQ¹Q², -C(O)NHOQ¹, -C(O)NH-NHQ¹, -C(O)O-NHQ¹,-(CH₂)ₘ-NHC(O)Q¹, -(CH₂)ₘ-NHS(O)₂Q¹, -(CH₂)ₘ-NHC(O)OQ¹, -(CH₂)ₘ-NHC(O)NQ¹Q², wherein the heterocycle is optionally substituted by one or more T¹, wherein m and T¹ are as defined above; or
- R¹ preferably represents -(CH₂)ₘNHQ³, -(CH₂)ₘ-NH-C(NHQ³)=NQ⁴, (CH₂)ₘ-NH-CH=NQ³, -C(NHQ³)=NQ⁴, wherein m is as defined above; or
- R¹ represents a carbon-linked 4-, 5- or 6-membered heterocycle saturated, partially or totally unsaturated or aromatic comprising at least one nitrogen atom, - (CH₂)mOC(O)Q¹, -C(O)OQ¹, -(CH₂)m-NHS(O)₂NQ¹Q², -(CH₂)ₘ-OC(O)OQ¹,-C(O)NHOQ¹, -C(O)NH-NHQ¹ ,-C(O)O-NHQ¹, -(CH₂)ₘ-NHC(O)Q¹, -(CH₂)ₘ-NHS(O)₂Q¹, -(CH₂)ₘ-NHC(O)OQ¹, -(CH₂)ₘ-NHC(O)NQ¹Q², -(CH₂)ₘ-NH-C(NHQ³)=NQ⁴, (CH₂)ₘ-NH-CH=NQ³ or -C(NHQ³)=NQ⁴, wherein the heterocycle is optionally substituted by one or more T¹, wherein m and T¹ are as defined above; or
- R¹ represents -(CH₂)ₘ-NH-C(NHQ³)=NQ⁴, (CH₂)ₘ-NH-CH=NQ³, -C(NHQ³)=NQ⁴, wherein m is as defined above ; or
- R¹ represents a carbon-linked 4-, 5- or 6-membered heterocycle saturated, partially or totally unsaturated or aromatic comprising at least one nitrogen atom, - (CH₂)ₘOC(O)Q¹, -C(O)OQ¹, -(CH₂)ₘ-OC(O)OQ¹, -(CH₂)ₘ-NHS(O)₂NQ¹Q²,-C(O)NHOQ¹, -C(O)NH-NHQ¹, -C(O)O-NHQ¹, -(CH₂)ₘ-NHC(O)Q¹, -(CH₂)ₘ NHS(O)₂Q¹, -(CH₂)ₘ-NHC(O)OQ¹, -(CH₂)ₘ-NHC(O)NQ¹Q² wherein the heterocycle is optionally substituted by one or more T¹ wherein m and T¹ are as defined above; and
- Q¹ and Q², identical or different independently represent H, methyl, -CH₂-CH₂-NH₂,-CH₂-CH₂-NH-CNH₂=NH, -CH₂-CH₂-NH-CH=NH, -CH₂-C(NH₂)=NH, CH₂-CH₂-OH,-CH₂-CONH₂, a -(CH₂)ₙ-heterocycle, wherein the heterocycle is a 4-, 5- or 6-membered heterocycle saturated, partially or totally unsaturated or aromatic comprising at least one nitrogen atom; wherein the heterocycle is independently optionally substituted by one or more T³, wherein n and T³ are as defined above; and
- Q³ and Q⁴ represent H.

Preferably, in the compounds of formula (I), (I*), (A), (A*), (B) and (B*):
- R² is a monocyclic or bicyclic 4- to 10-membered heterocycle, saturated, partially or totally unsaturated or aromatic, optionally substituted by one or more T²; and
- R¹ represents a carbon-linked 4-, 5- or 6-membered heterocycle saturated, partially or totally unsaturated or aromatic comprising at least one nitrogen atom, -CN, -(CH₂)ₘ-OQ¹, -(CH₂)m-OC(O)Q¹, -C(O)OQ¹, -(CH₂)ₘ-OC(O)OQ¹, -(CH₂)ₘ-OC(O)NQ¹Q², - C(O)NHQ¹, -(CH₂)ₘ-NHS(O)₂NQ¹Q², -C(O)NHOQ¹, -C(O)NH-NHQ¹, -C(O)O-NHQ¹,-(CH₂)ₘ-NHC(O)Q¹, -(CH₂)ₘ-NHS(O)₂Q¹, -(CH₂)ₘ-NHC(O)OQ¹, -(CH₂)ₘ-NHC(O)NQ¹Q², wherein the heterocycle is optionally substituted by one or more T¹, wherein m and T¹ are as defined above; or
- R¹ represents -(CH₂)ₘNHQ³, -(CH₂)ₘ-NH-C(NHQ³)=NQ⁴, (CH₂)ₘ-NH-CH=NQ³,-C(NHQ³)=NQ⁴, wherein m is as defined above; or
- R¹ represents a carbon-linked 4-, 5- or 6-membered heterocycle saturated, partially or totally unsaturated or aromatic comprising at least one nitrogen atom, - (CH₂)ₘOC(O)Q¹, -C(O)OQ¹, -(CH₂)m-NHS(O)2NQ¹Q², -(CH₂)ₘ-OC(O)OQ¹, - C(O)NHOQ¹, -C(O)NH-NHQ¹ ,-C(O)O-NHQ¹, -(CH₂)ₘ-NHC(O)Q¹, -(CH₂)ₘNHS(O)₂Q¹, -(CH₂)ₘ-NHC(O)OQ¹, -(CH₂)ₘ-NHC(O)NQ¹Q², -(CH₂)ₘ-NH-C(NHQ³)=NQ⁴, (CH₂)ₘ-NH-CH=NQ³ or -C(NHQ³)=NQ⁴, wherein the heterocycle is optionally substituted by one or more T¹, wherein m and T¹ are as defined above; or
- R¹ represents -(CH₂)ₘ-NH-C(NHQ³)=NQ⁴, (CH₂)ₘ-NH-CH=NQ³, -C(NHQ³)=NQ⁴, wherein m is as defined above ; or
- R¹ represents a carbon-linked 4-, 5- or 6-membered heterocycle saturated, partially or totally unsaturated or aromatic comprising at least one nitrogen atom, - (CH₂)ₘOC(O)Q¹, -C(O)OQ¹, -(CH₂)ₘ-OC(O)OQ¹, -(CH₂)ₘ-NHS(O)₂NQ¹Q², -C(O)NHOQ¹, -C(O)NH-NHQ¹, -C(O)O-NHQ¹, -(CH₂)ₘ-NHC(O)Q¹, - (CH₂)ₘNHS(O)₂Q¹, -(CH₂)ₘ-NHC(O)OQ¹, -(CH₂)ₘ-NHC(O)NQ¹Q² wherein the heterocycle is optionally substituted by one or more T¹ wherein m and T¹ are as defined above; and
- Q¹ and Q², identical or different independently represent H, methyl, -CH₂-CH₂-NH₂,-CH₂-CH₂-NH-CNH₂=NH, -CH₂-CH₂-NH-CH=NH, -CH₂-C(NH₂)=NH, CH₂-CH₂-OH,-CH₂-CONH₂, a -(CH₂)ₙ-heterocycle, wherein the heterocycle is a 4-, 5- or 6-membered heterocycle saturated, partially or totally unsaturated or aromatic comprising at least one nitrogen atom; wherein the heterocycle is independently optionally substituted by one or more T³, wherein n and T³ are as defined above; and
- Q³ and Q⁴ represent H; and
- R³ represent SO₃H or CF₂COOH.

Preferably, in the compounds of formula (I), (I*), (A), (A*), (B) and (B*):
- R² is a monocyclic or bicyclic 4- to 10-membered heterocycle, saturated, partially or totally unsaturated or aromatic, optionally substituted by one or more T²; and
- R¹ represents a carbon-linked 4-, 5- or 6-membered heterocycle saturated, partially or totally unsaturated or aromatic comprising at least one nitrogen atom, CN, - C(O)NHQ¹, -C(O)NHOQ¹, -C(O)NH-NHQ¹ or -(CH₂)ₘOQ¹ wherein the heterocycle is optionally substituted by one or more T¹, wherein m, Q¹ and T¹ are as defined above; or
- R¹ represents -(CH₂)ₘNHQ³, -(CH₂)ₘ-NH-C(NHQ³)=NQ⁴, wherein m, Q³ and Q⁴ are as defined above; or
- R¹ represents -C(NHQ³)=NQ⁴, wherein m, Q³ and Q⁴ are as defined above; or
- R¹ represents a carbon-linked 4-, 5- or 6-membered heterocycle saturated, partially or totally unsaturated or aromatic comprising at least one nitrogen atom, -C(O)NHOQ¹ or -C(O)NH-NHQ¹, wherein the heterocycle is optionally substituted by one or more T¹, wherein m, Q¹ and T¹ are as defined above.

Preferably, in the compounds of formula (I), (I*), (A), (A*), (B) and (B*):
- R² is a monocyclic or bicyclic 4- to 10-membered heterocycle, saturated, partially or totally unsaturated or aromatic, optionally substituted by one or more T²; and
- R¹ represents a carbon-linked 4-, 5- or 6-membered heterocycle saturated, partially or totally unsaturated or aromatic comprising at least one nitrogen atom, CN, - C(O)NHQ¹, -C(O)NHOQ¹, -C(O)NH-NHQ¹ or -(CH₂)ₘOQ¹ wherein the heterocycle is optionally substituted by one or more T¹, wherein m and T¹ are as defined above; or
- R¹ represents -(CH₂)ₘNHQ³, -(CH₂)ₘ-NH-C(NHQ³)=NQ⁴, wherein m is as defined above; or
- R¹ represents -C(NHQ³)=NQ⁴, wherein m is as defined above; or
- R¹ represents a carbon-linked 4-, 5- or 6-membered heterocycle saturated, partially or totally unsaturated or aromatic comprising at least one nitrogen atom, -C(O)NHOQ¹ or -C(O)NH-NHQ¹, wherein the heterocycle is optionally substituted by one or more T¹, wherein m and T¹ are as defined above; and
- Q¹ independently represents H, methyl, -CH₂-CH₂-NH₂, -CH₂-CH₂-NH-CNH₂=NH,-CH₂-CH₂-NH-CH=NH, -CH₂-C(NH₂)=NH, CH₂-CH₂-OH, -CH₂-CONH₂, a -(CH₂)ₙ-heterocycle, wherein the heterocycle is a 4-, 5- or 6-membered heterocycle saturated, partially or totally unsaturated or aromatic comprising at least one nitrogen atom; wherein the heterocycle is independently optionally substituted by one or more T³, wherein n and T³ are as defined above; and
- Q³ and Q⁴ represent H.

Preferably, in the compounds of formula (I), (I*), (A), (A*), (B) and (B*):
- R² is a monocyclic or bicyclic 4- to 10-membered heterocycle, saturated, partially or totally unsaturated or aromatic, optionally substituted by one or more T²; and
- R¹ represents a carbon-linked 4-, 5- or 6-membered heterocycle saturated, partially or totally unsaturated or aromatic comprising at least one nitrogen atom, CN, - C(O)NHQ¹, -C(O)NHOQ¹, -C(O)NH-NHQ¹ or -(CH₂)ₘOQ¹ wherein the heterocycle is optionally substituted by one or more T¹, wherein m and T¹ are as defined above; or
- R¹ represents -(CH₂)ₘNHQ³, -(CH₂)ₘ-NH-C(NHQ³)=NQ⁴, wherein m is as defined above; or
- R¹ represents -C(NHQ³)=NQ⁴, wherein m is as defined above; or
- R¹ represents a carbon-linked 4-, 5- or 6-membered heterocycle saturated, partially or totally unsaturated or aromatic comprising at least one nitrogen atom, -C(O)NHOQ¹ or -C(O)NH-NHQ¹, wherein the heterocycle is optionally substituted by one or more T¹, wherein m and T¹ are as defined above; and
- Q¹ independently represents H, methyl, -CH₂-CH₂-NH₂, -CH₂-CH₂-NH-CNH₂=NH,-CH₂-CH₂-NH-CH=NH, -CH₂-C(NH₂)=NH, CH₂-CH₂-OH, -CH₂-CONH₂, a -(CH₂)ₙ-heterocycle, wherein the heterocycle is a 4- to 6-membered heterocycle saturated, partially or totally unsaturated or aromatic comprising at least one nitrogen atom; wherein the heterocycle is independently optionally substituted by one or more T³, wherein n and T³ are as defined above; and
- Q³ and Q⁴ represent H; and
- R³ represent SO₃H or CF₂COOH.

Preferably in the compounds of formula (I), (I*), (A), (A*), (B) and (B*):
- R² is a monocyclic or bicyclic 4- to 10-membered heterocycle, saturated, partially or totally unsaturated or aromatic, optionally substituted by one or more T²; and
- R¹ represents a carbon-linked 4-, 5- or 6-membered heterocycle saturated, partially or totally unsaturated or aromatic comprising at least one nitrogen atom, optionally substituted by one or more T¹; -C(O)NHQ¹, -(CH₂)ₘ-NH-C(NHQ³)=NQ⁴ or-(CH₂)ₘNHQ³, wherein Q¹, Q³, Q⁴, T¹ and m are as defined above.

Preferably in the compounds of formula (I), (I*), (A), (A*), (B) and (B*):
- R² is a monocyclic or bicyclic 4- to 10-membered heterocycle, saturated, partially or totally unsaturated or aromatic, optionally substituted by one or more T²; and
- R¹ represents a carbon-linked 4-, 5- or 6-membered heterocycle saturated, partially or totally unsaturated or aromatic comprising at least one nitrogen atom, optionally substituted by one or more T¹; -C(O)NHQ¹, -(CH₂)ₘ-NH-C(NHQ³)=NQ⁴ or-(CH₂)ₘNHQ³, wherein T¹ and m are as defined above; and
- Q¹ independently represents H, methyl, -CH₂-CH₂-NH₂, -CH₂-CH₂-NH-CNH₂=NH,-CH₂-CH₂-NH-CH=NH, -CH₂-C(NH₂)=NH, CH₂-CH₂-OH, -CH₂-CONH₂, a -(CH₂)ₙ-heterocycle, wherein the heterocycle is a 4-, 5- or 6-membered heterocycle saturated, partially or totally unsaturated or aromatic comprising at least one nitrogen atom; wherein the heterocycle is independently optionally substituted by one or more T³, wherein n and T³ are as defined above; and
- Q³ and Q⁴ represents H.

Preferably in the compounds of formula (I), (I*), (A), (A*), (B) and (B*):
- R² is a monocyclic or bicyclic 4- to 10-membered heterocycle, saturated, partially or totally unsaturated or aromatic, optionally substituted by one or more T²; and
- R¹ represents a carbon-linked 4-, 5- or 6-membered heterocycle saturated, partially or totally unsaturated or aromatic comprising at least one nitrogen atom, optionally substituted by one or more T¹; -C(O)NHQ¹, -(CH₂)ₘ-NH-C(NHQ³)=NQ⁴ or-(CH₂)ₘNHQ³, wherein T¹ and m are as defined above; and
- Q¹ independently represents H, methyl, -CH₂-CH₂-NH₂, -CH₂-CH₂-NH-CNH₂=NH,-CH₂-CH₂-NH-CH=NH, -CH₂-C(NH₂)=NH, CH₂-CH₂-OH, -CH₂-CONH₂, a -(CH₂)ₙ-heterocycle, wherein the heterocycle is a 4-, 5- or 6-membered heterocycle saturated, partially or totally unsaturated or aromatic comprising at least one nitrogen atom; wherein the heterocycle is independently optionally substituted by one or more T³, wherein n and T³ are as defined above; and
- Q³ and Q⁴ represents H; and
- R³ represent SO₃H or CF₂COOH.

Preferably in the compounds of formula (I), (I*), (A), (A*), (B) and (B*):
- R² is a monocyclic or bicyclic 4- to 10-membered heterocycle, saturated, partially or totally unsaturated or aromatic, optionally substituted by one or more T²; and
- Preferably, R¹ represents (CH₂)NH₂ or (CH₂)-NH-C(NH₂)=NH.

Preferably in the compounds of formula (I), (I*), (A), (A*), (B) and (B*):
- R² is a monocyclic or bicyclic 4- to 10-membered heterocycle, saturated, partially or totally unsaturated or aromatic, optionally substituted by one or more T²; and
- Preferably, R¹ represents (CH₂)NH₂ or (CH₂)-NH-C(NH₂)=NH; and
- R³ represent SO₃H or CF₂COOH.

The term "alkyl", as used herein, refers to an aliphatic-hydrocarbon group which may be straight or branched, having 1 to 3 carbon atoms in the chain unless specified otherwise. Preferred alkyl groups have 1 or 2 carbon atoms in the chain. Specific examples of alkyl groups include, but are not limited to, methyl, ethyl, n-propyl, iso propyl. Preferably, the alkyl group is methyl or ethyl.

The term "fluoroalkyl", as used herein, refers to an alkyl group substituted with at least one fluorine atom. The term "alkyl" is as defined above. Specific examples of fluoroalkyl groups include but are not limited to trifluoromethyl, difluoromethyl, fluoromethyl.

The term "cycloalkyl" refers to a saturated monocyclic or bicyclic non-aromatic hydrocarbon ring of 3 to 6 carbon atoms, preferably 3 to 4 carbon atoms, which can comprise one or more unsaturation. Specific examples of monocyclic cycloalkyl groups include cyclopropyl, cyclobutyl, cyclopentyl, cyclohexyl. Preferably, the cycloalkyl group is cyclopropyl or cyclobutyl.

The term "fluorocycloalkyl" refers to a cycloalkyl group substituted with at least one fluorine atom. The term "cycloalkyl" is as defined above. Specific examples of fluorocycloalkyl groups include fluorocyclopropyl, difluorocyclopropyl, fluorocyclobutyl, difluorocyclobutyl.

The term "heterocycle", as used herein and without contrary definition specifically mentioned, either alone or in combination with another radical, refers to a monocyclic or bicyclic saturated, partially or totally unsaturated or aromatic hydrocarbon radical, preferably 4 to 10-membered, comprising at least one heteroatom, such as N, O, S, S(O) or S(O)₂. Preferably, the heterocycle is a monocyclic saturated, partially or totally unsaturated or aromatic hydrocarbon radical, preferably 4, 5- or 6-membered, comprising at least one nitrogen atom and which can comprise at least one further heteroatom, such as N, O, S, S(O) or S(O)₂, the carbon atoms of the heterocycle can also be oxidized as C(O). Suitable heterocycles are also disclosed in the Handbook of Chemistry and Physics, 76th Edition, CRC Press, Inc., 1995-1996, pages 2 25 to 2-26. Examplary heterocycle groups include, but are not limited to, azetidinyl, oxetanyl, oxazolyl, oxazolidinyl, oxadiazolyl, pyrrolyl, pyrrolidinyl, pyridyl, tetrahydropyridinyl, piperidinyl, morpholinyl, pyrazolyl, pyrimidinyl, pyrazinyl, tetrazolyl, imidazolyl, thienyl, thiazolyl, furanyl, thiadiazolyl, isothiazolyl, triazolyl, tetrazolyl, pyrazolyl, isoxazolyl, 2-pyrrolidinonyl, imidazol-2,4-dione, 1,2,4-oxadiazolyl-5-one, 1,5-dihydropyrrolyl-2-one, pyrazinone, pyridazinone, pyridone, pyrimidone, dioxanyl, pyrrolidinyl, imidazolidinyl, pyranyl, tetrahydrofuranyl, dioxolanyl, tetrahydropyranyl. Preferably, in the compounds according to the invention, the heterocycle is linked to the structure of the compounds by a carbon atom of the heterocycle (also said carbon-linked heteroatom).

Moreover some compounds according to this invention may contain a basic amino group and thus may form an inner zwitterionic salt (or zwitterion) with the acidic group (R³) -OSO₃H, - OCFHCO₂H or -OCF₂CO₂H and such inner zwitterionic salts are also included in this invention.

The term "optionally substituted" means "non-substituted or substituted".

The term "racemate" is employed herein to refer to an equal amount of two specific enantiomers.

The term "enantiomer" is employed herein to refer to one of the two specific stereoisomers which is a non-superimposable mirror image with one other but is related to one other by reflection.

The compounds of the invention can possess one or more asymmetric carbon atoms and are thus capable of existing in the form of optical isomers as well as in the form of racemic or non-racemic mixtures thereof. The compounds of the invention can be used in the present invention as a single isomer or as a mixture of stereochemical isomeric forms. Diastereoisomers, i.e., nonsuperimposable stereochemical isomers can be separated by conventional means such as chromatography, distillation, crystallization or sublimation. The optical isomers (enantiomers) can be obtained by using optically active starting materials, by resolution of the racemic mixtures according to conventional processes, for example by formation of diastereoisomeric salts by treatment with an optically active acid or base or by using chiral chromatography column.

The expression "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, the expression "pharmaceutically acceptable salts" refers to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts of the present invention can be synthesized from the parent compound which comprises a basic or an acidic moiety, by conventional chemical methods. Furthermore, the expression "pharmaceutically acceptable salt" refers to relatively non-toxic, inorganic and organic acid or base addition salts of the compounds of the present invention. These salts can be prepared in situ during the final isolation and purification of the compounds. In particular, the acid addition salts can be prepared by separately reacting the purified compound in its purified form with an organic or inorganic acid and by isolating the salt thus formed. Among the examples of acid addition salts are the hydrobromide, hydrochloride, hydroiodide, sulfamate, sulfate, bisulfate, phosphate, nitrate, acetate, propionate, succinate, oxalate, valerate, oleate, palmitate, stearate, laurate, borate, benzoate, lactate, tosylate, citrate, maleate, fumarate, tartrate, naphthylate, mesylate, glucoheptanate, glucoronate, glutamate, lactobionate, malonate, salicylate, methylenebis-b-hydroxynaphthoate, gentisic acid, isethionate, di-p-toluoyltartrate, ethanesulfonate, benzenesulfonate, cyclohexyl sulfamate, quinateslaurylsulfonate salts, and the like. Examples of base addition salts include ammonium salts such as tromethamine, meglumine, epolamine, etc, metal salts such as sodium, lithium, potassium, calcium, zinc or magnesium salts with organic bases such as dicyclohexylamine salts, N-methyl-D-glucamine. Lists of suitable salts may be found in Remington's Pharmaceutical Sciences, 17th ed., Mack Publishing Company, Easton, PA, 1985, p. 1418, P.H. Stahl, C.G. Wermuth, Handbook of Pharmaceutical salts - Properties, Selection and Use, Wiley-VCH, 2002 and S.M. Berge et al. "Pharmaceutical Salts" J. Pharm. Sci, 66: p.1-19 (1977).

Compounds according to the invention also include isotopically-labeled compounds wherein one or more atoms is replaced by an atom having the same atomic number, but an atomic mass or mass number different from the atomic mass or mass number usually found in nature. Examples of isotopes suitable for inclusion in the compounds described above and are not limited to ²H, ³H, ¹¹C, ¹³C, ¹⁴C, ¹⁹F, ¹⁸F, ¹⁵N, ¹³N, ³³S, ³⁴S, ³⁵S, ³⁶S, ¹⁷O or ¹⁸O. In one embodiment, isotopically-labeled compounds are useful in drug and/or substrate tissue distribution studies. In another embodiment, substitution with heavier isotopes such as deuterium (²H) affords greater metabolic stability (for example increased in vivo half-life or reduced dosage requirements). Isotopically-labeled compounds are prepared by any suitable method or by processes using an appropriate isotopically-labeled reagent in place of the non-labeled reagent otherwise employed.

The present invention also relates to a composition, preferably a pharmaceutical composition, comprising at least one compound of formula (I), (I*), (A), (A*), (B) or (B*) as defined above, with a pharmaceutically acceptable excipient.

The composition according to the invention can further comprise at least one or more antibacterial agent(s), preferably at least one of these antibacterial agents is a beta-lactam.

The term "beta-lactam" or "β-lactam" refers to antibacterial compounds comprising a β-lactam unit, i.e. a group.

The term "pharmaceutically acceptable carrier" or "pharmaceutically acceptable excipient" is employed for any excipient, solvent, dispersion medium, absorption retardant, diluent or adjuvant etc., such as preserving or antioxidant agents, fillers, binders, disintegrating agents, wetting agents, emulsifying agents, suspending agents, solvents, dispersion media, coatings, antibacterial agents, isotonic and absorption delaying agents and the like, that does not produce a secondary reaction, for example an allergic reaction, in humans or animals. Typical, non-limiting examples of excipients include mannitol, lactose, magnesium stearate, sodium saccharide, talcum, cellulose, sodium croscarmellose, glucose, gelatin, starch, lactose, dicalcium phosphate, sucrose, kaolin, magnesium carbonate, wetting agents, emulsifying agents, solubilizing agents, sterile water, saline, pH buffers, non-ionic surfactants, lubricants, stabilizing agents, binding agents and edible oils such as peanut oil, sesame oils and the like. In addition, various excipients commonly used in the art may be included. Pharmaceutically acceptable carriers or excipients are well known to a person skilled in the art, and include those described in Remington's Pharmaceutical Sciences (Mack Publishing Company, Easton, USA, 1985), Merck Index (Merck & Company, Rahway, N.J.), Gilman et al (Eds. The pharmacological basis of therapeutics, 8th Ed., pergamon press., 1990). Except insofar as any conventional media or adjuvant is incompatible with the active ingredient according to the invention, its use in the therapeutic compositions is contemplated.

The expression "antibacterial agent" as used herein, refers to any substance, compound or their combination capable of inhibiting, reducing or preventing growth of bacteria, inhibiting or reducing ability of bacteria to produce infection in a subject, or inhibiting or reducing ability of bacteria to multiply or remain infective in the environment, or decreasing infectivity or virulence of bacteria.

The antibacterial agent is selected among the following families: aminoglycosides, beta-lactams, glycylcyclines, tetracyclines, quinolones, fluoroquinolones, glycopeptides, lipopeptides, macrolides, ketolides, lincosamides, streptogramins, oxazolidinones and polymyxins alone or in mixture.
Preferably, the further antibacterial agent is selected among the beta-lactam families, and more preferably among penicillin, cephalosporins, penems, carbapenems and monobactam, alone or in mixture.
Among the penicillin the antibacterial agent is preferably selected in the group consisting of amoxicillin, ampicillin, azlocillin, mezocillin, apalcillin, hetacillin, bacampicillin, carbenicillin, sulbenicillin, temocillin, ticarcillin, piperacillin, mecillinam, pivmecillinam, methicillin, ciclacillin, talampacillin, aspoxicillin, oxacillin, cloxacillin, dicloxacillin, flucloxacillin, nafcillin, and pivampicillin, alone or in mixture.
Among the cephalosporin, the antibacterial agent is preferably selected in the group consisting of cefatriazine, cefazolin, cefoxitin, cephalexin, cephradine, ceftizoxime, cephacetrile, cefbuperazone, cefprozil, ceftobiprole, ceftobiprole medocaril, ceftaroline, ceftaroline fosaminyl, cefalonium, cefminox, ceforanide, cefotetan, ceftibuten, cefcapene pivoxil, cefditoren pivoxil, cefdaloxime cefroxadine, ceftolozane and S-649266, cephalothin, cephaloridine, cefaclor, cefadroxil, cefamandole, cefazolin, cephalexin, cephradine, ceftizoxime, cephacetrile, cefotiam, cefotaxime, cefsulodin, cefoperazone, cefmenoxime, cefmetazole, cephaloglycin, cefonicid, cefodizime, cefpirome, ceftazidime, ceftriaxone, cefpiramide, cefbuperazone, cefozopran, cefepime, cefoselis, cefluprenam, cefuzonam, cefpimizole, cefclidine, cefixime, ceftibuten, cefdinir, cefpodoxime axetil, cefpodoxime proxetil, cefteram pivoxil, cefetamet pivoxil, cefcapene pivoxil, cefditoren pivoxil, cefuroxime, cefuroxime axetil, loracarbef, and latamoxef, alone or in mixture.
Among the carbapenem, the antibacterial agent is preferably selected in the group consisting of imipenem, doripenem, meropenem, biapenem, ertapenem and panipenem, alone or in mixture.
Among the monobactam the antibacterial agent is preferably selected in the group consisting of aztreonam, tigemonam, carumonam, BAL30072 and nocardicin A, alone or in mixture.

Preferably, the invention relates to a pharmaceutical composition comprising
- a single compound of formula (I), (I*), (A), (A*), (B) and (B*) ;
- a compound of formula (I), (I*), (A), (A*), (B) and (B*) and one or more antibacterial compound ;
- a compound of formula (I), (I*), (A), (A*), (B) and (B*) and one or more β-lactam compound ;
- a compound of formula (I), (I*), (A), (A*), (B) and (B*), one or more antibacterial compound and one or more β-lactam compound.

The present invention also relates to a kit comprising:
- a pharmaceutical composition according to the invention, and
- at least one other composition comprising one or more antibacterial agent(s), preferably at least one of these antibacterial agent(s) is a beta-lactam.

The two composition can be prepared separately each with one specific pharmaceutically acceptable carrier, and can be mix especially extemporaneity.

The present invention also refer to a compound of formula (I), (I*), (A), (A*), (B) or (B*) according to the invention for use as a medicine.

The present invention also refer to the use of a compound of formula (I), (I*), (A), (A*), (B) or (B*) according to the invention or of a composition according to the invention for the preparation of a medicine.

The present invention also provides the use of the compounds of formula (I), (I*), (A), (A*), (B) and (B*) on the control of bacteria. The compound according to the invention is usually used in combination with pharmaceutically acceptable excipient.

The present invention also refer to a compound of formula (I), (I*), (A), (A*), (B) or (B*) according to the invention for use as antibacterial agent.

The present invention also refer to the use of a compound of formula (I), (I*), (A), (A*), (B) or (B*) according to the invention or of a composition according to the invention for the preparation of an antibacterial agent medicine.

The present invention also refer to the use of a compound of formula (I), (I*), (A), (A*), (B) or (B*) according to the invention or of a composition according to the invention for the preparation of an inhibitor of beta-lactamase medicine.

The present invention also refer to the use of a compound of formula (I), (I*), (A), (A*), (B) or (B*) according to the invention or of a composition according to the invention for the preparation of an antibacterial agent and inhibitor of beta-lactamase medicine.

The present invention also refer to a compound of formula (I), (I*), (A), (A*), (B) or (B*) or a composition according to the invention or a kit according to the invention for use for the treatment or prevention of bacterial infections.

The present invention also refer to the use of a compound of formula (I), (I*), (A), (A*), (B) or (B*) or a composition according to the invention for the preparation of a medicine for the treatment or prevention of bacterial infections.

The terms "prevention", "prevent" and "preventing" as used herein are intended to mean the administration of a compound or composition according to the invention in order to prevent infection by bacteria or to prevent occurrence of related infection and/or diseases. The terms "prevention", "prevent" and "preventing" also encompass the administration of a compound or composition according to the present invention in order preventing at least one bacterial infection, by administration to a patient susceptible to be infected, or otherwise at a risk of infection by this bacteria.

The terms "treatment", "treat" and "treating" as used herein are intended to mean in particular the administration of a treatment comprising a compound or composition according to the present invention to a patient already suffering from an infection. The terms "treatment", "treat" and "treating" as used herein, also refer to administering a compound or composition according to the present invention, optionally with one or more antibacterial agent, in order to:
- reduce or eliminate either a bacterial infection or one or more symptoms associated with bacterial infection, or
- retard the progression of a bacterial infection or of one or more symptoms associated with bacterial infection, or
- reduce the severity of a bacterial infection or of one or more symptoms associated with the bacterial infection, or
- suppress the clinical manifestation of a bacterial infection, or
- suppress the manifestation of adverse symptoms of the bacterial infection.

The expression "infection" or "bacterial infection" as used herein, includes the presence of bacteria, in or on a subject, which, if its growth were inhibited, would result in a benefit to the subject. As such, the term "infection" or "bacterial infection" in addition to referring to the presence of bacteria also refers to normal flora, which is not desirable. The term "infection" includes infection caused by bacteria. Exemplary of such bacterial infection are urinary tract infection (UTI), kidney infections (pyelonephritis), gynecological and obstetrical infections, respiratory tract indection (RTI), acute exacerbation of chronic bronchitis (AECB), Community-acquired pneumonia (CAP), hospital-acquired pneumonia (HAP), ventilator associated pneumonia (VAP), intra-abdominal pneumonia (IAI), acute otitis media, acute sinusitis, sepsis, catheter-related sepsis, chancroid, chlamydia, skin infections, bacteremia.

The term "growth" as used herein, refers to the growth of one or more microorganisms and includes reproduction or population expansion of the microorganism, such as bacteria. The term also includes maintenance of on-going metabolic processes of a microorganism, including processes that keep the microorganism alive.

The bacteria are chosen amongst gram-positive bacteria or gram-negative bacteria, preferably the gram-negative bacteria.

The bacteria can be also chosen among bacteria producing "beta-lactamase" or "β-lactamase". These bacteria are well known by the skilled person.

The term "beta-lactamase" or "β-lactamase" as used herein, refers to any enzyme or protein or any other substance that is able to break down a beta-lactam ring. The term "beta-lactamase" or "β-lactamase" includes enzymes that are produced by bacteria and that have the ability to hydrolyze, either partially or completely, the beta-lactam ring present in a compound such as an antibacterial agent.

Among the gram-positive bacteria, the bacteria according to the invention is preferably chosen among *Staphylococcus, Streptococcus, Staphylococcus species* (including *Staphylococcus aureus, Staphylococcus epidermidis), Streptococcus species* (including *Streptococcus pneumonia, Streptococcus agalactiae), Enterococcus species* (including *Enterococcus faecalis* and *Enterococcus faecium*).

Among the gram-negative bacteria, the bacteria according to the invention is preferably chosen among *Acinetobacter* species (including *Acinetobacter baumannii*), *Citrobacter* species, *Escherichia* species (including *Escherichia coli*), *Haemophilus influenza, Morganella morganii, Klebsiella* species (including *Klebsiella pneumonia), Enterobacter* species (including *Enterobacter cloacae), Neisseria gonorrhoeae, Burkholderia* species (including *Burkholderia cepacia), (Proteus* species (including *Proteus mirabilis*), *Serratia* species (including *Serratia marcescens), Pseudomonas aeruginosa.*

The invention thus preferably refers to a compound of formula (I), (I*), (A), (A*), (B) or (B*), or a composition according to the invention or a kit according to the invention for use for the treatment or prevention of bacterial infection, preferably caused by bacteria producing one or more beta-lactamase(s). Preferably, the bacteria are chosen amongst gram-positive bacteria or gram-negative bacteria, preferably gram-negative bacteria.

The present invention also refer to the use of a compound of formula (I), (I*), (A), (A*), (B) or (B*) or a composition according to the invention for the preparation of a medicine for the treatment or prevention of bacterial infection, preferably caused by bacteria producing one or more beta-lactamase(s). Preferably, the bacteria are chosen amongst gram-positive bacteria or gram-negative bacteria, preferably gram-negative bacteria.

The present invention also refers to the kit as defined above, for a simultaneous, separated or sequential administration to a patient in need thereof for use for the treatment or prevention of bacterial infections, preferably caused by bacteria producing one or more beta-lactamase(s). Preferably, the bacteria are chosen amongst gram-positive bacteria or gram-negative bacteria, preferably gram-negative bacteria.

The present invention also refers to compound of formula (I), (I*), (A), (A*), (B) or (B*) for use in combination with one or more further antibacterial agent, preferably at least one of the further antibacterial agent is a beta lactam, for the treatment or prevention of bacterial infections, preferably caused by bacteria producing one or more beta-lactamase(s). Preferably, the bacteria are chosen amongst gram-positive bacteria or gram-negative bacteria, preferably gram-negative bacteria. Wherein the compounds of formula (I), (I*), (A), (A*), (B) or (B*) and the further antibacterial agent are administered simultaneously, separately or sequentially.

The present invention also refers to the use of a compound of formula (I), (I*), (A), (A*), (B) or (B*) or a composition according to the invention or a kit according to the invention for the prevention or treatment of bacterial infections, preferably of bacterial infection, preferably caused by bacteria producing one or more beta-lactamase(s). Preferably, the bacteria are chosen amongst gram-positive bacteria or gram-negative bacteria, preferably gram-negative bacteria.

The present invention also relates to a method for the treatment or prevention of bacterial infections, preferably caused by bacteria producing one or more beta-lactamase(s) comprising the administration of a therapeutically effective amount of compound of formula (I), (I*), (A), (A*), (B) or (B*), a composition according to the invention or a kit according to the invention to a patient in need thereof. Preferably, the bacteria are chosen amongst gram-positive bacteria or gram-negative bacteria, preferably gram-negative bacteria.

The term "patient" means a person or an animal at risk of being infected by bacteria or, a person or an animal being infected by bacteria, preferably by gram-positive and/or by gram-negative bacteria. As used herein, the term "patient" refers to a warm-blooded animal such as a mammal, preferably a human or a human child, who is afflicted with, or has the potential to be afflicted with one or more infections and conditions described herein. The identification of those subjects who are in need of treatment of herein-described diseases and conditions is well within the ability and knowledge of one skilled in the art. A veterinarian or a physician skilled in the art can readily identify, by the use of clinical tests, physical examination, medical/family history or biological and diagnostic tests, those subjects who are in need of such treatment.

The expression "therapeutically effective amount" or "pharmaceutically effective amount" as used herein, refer to an amount of a compound according to the invention, which when administered to a patient in need thereof, is sufficient to effect treatment for disease-states, conditions, or disorders for which the compound has utility. Such an amount would be sufficient to elicit the biological or medical response of a tissue system, or patient that is sought by a researcher or a clinician. The amount of a compound according to the invention which constitutes a "therapeutically effective amount" will vary, notably depending on the compound itself and its biological activity, the composition used for administration, the time of administration, the route of administration, the rate of excretion of the compound, the duration of the treatment, the type of disease-state or disorder being treated and its severity, drugs used in combination with or coincidentally with the compounds of the invention, and the age, body weight, general health, sex and diet of the patient. Such a "therapeutically effective amount" can be determined by one of ordinary skilled in the art having regard to its own knowledge, and this disclosure. Preferably, the compounds according to the invention are administered in an amount comprised between 0.1 to 30g per day.

The compounds according to the invention may be provided in an aqueous physiological buffer solution for parenteral administration.
The compounds of the present invention are also capable of being administered in unit dose forms, wherein the expression "unit dose" means a single dose which is capable of being administered to a patient, and which can be readily handled and packaged, remaining as a physically and chemically stable unit dose comprising either the active compound itself, or as a pharmaceutically acceptable composition, as described hereinafter. Compounds provided herein can be formulated into pharmaceutical compositions by admixture with one or more pharmaceutically acceptable excipients. Such unit dose compositions may be prepared for use by oral administration, particularly in the form of tablets, simple capsules or soft gel capsules; or intranasally, particularly in the form of powders, nasal drops, or aerosols; or dermally, for example, topically in ointments, creams, lotions, gels or sprays, or via trans-dermal patches. The compositions may conveniently be administered in unit dosage form and may be prepared by any of the methods well-known in the pharmaceutical art, for example, as described in Remington: The Science and Practice of Pharmacy, 20th ed.; Gennaro, A. R., Ed.; Lippincott Williams & Wilkins: Philadelphia, PA, 2000.
Preferred formulations include pharmaceutical compositions in which a compound of the present invention is formulated for oral or parenteral administration.
For oral administration, tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolved, modified-release or sustained-release preparations and formulations, and wherein such sustained-release formulations are preferably bi-modal. Preferred tablets contain lactose, cornstarch, magnesium silicate, croscarmellose sodium, povidone, magnesium stearate, or talc in any combination.
Liquid preparations for parenteral administration include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, vegetable oils such as olive oil, and organic esters such as ethyl oleate. Aqueous carriers include mixtures of alcohols and water, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like. Other potentially useful parenteral delivery systems for these active compounds include ethylene-vinyl acetate copolymer particles, osmotic pumps, implantable infusion systems, and liposomes.
Alternative modes of administration include formulations for inhalation, which include such means as dry powder, aerosol, or drops. They may be aqueous solutions containing, for example, polyoxyethylene-9-lauryl ether, glycocholate and deoxycholate, or oily solutions for administration in the form of nasal drops, or as a gel to be applied intranasally. Formulations for buccal administration include, for example, lozenges or pastilles and may also include a flavored base, such as sucrose or acacia, and other excipients such as glycocholate. Formulations suitable for rectal administration are preferably presented as unit-dose suppositories, with a solid based carrier, and may include a salicylate. Formulations for topical application to the skin preferably take the form of an ointment, cream, lotion, paste, gel, spray, aerosol, or oil. Carriers which can be used include petroleum jelly, lanolin, polyethylene glycols, alcohols, or their combinations. Formulations suitable for transdermal administration can be presented as discrete patches and can be lipophilic emulsions or buffered, aqueous solutions, dissolved and/or dispersed in a polymer or an adhesive.

The present invention also relates to process for the preparation of compounds of formula (I), (I*), (A), (A*), (B) and (B*) as defined above.

Preparation of the compounds: Abbreviations or symbols used herein include:
- ACHN:: 1,1'-azobis(cyclohexanecarbonitrile)
- ACN:: acetonitrile
- AcOH:: acetic acid
- Bn:: benzyl
- Boc:: *tert*-butoxycarbonyl
- Boc₂O:: *tert*-butoxycarbonyl anhydride
- bs:: broad singlet
- CFU:: colony-forming units
- CLSI:: clinical laboratory standards institute
- d:: doublet
- DBU:: 1,8-diazabicyclo[5.4.0]undec-7-ene
- DCM:: dichloromethane
- dd:: doubled doublet
- ddd :: doubled doubled doublet
- dt :: doubled triplet
- DTA:: di-*tert*-butylazodicarboxylate
- DEAD:: diethyl azodicarboxylate
- DIAD:: diisopropyl azodicarboxylate
- DIPEA:: diisopropylethylamine
- DMF:: *N*,*N*-dimethylformamide
- DMAP:: 4-dimethylaminopyridine
- DMSO:: dimethylsulfoxide
- EtOAc:: ethyl acetate
- Et₂O:: diethyl ether
- h:: hours
- m :: massif
- min:: minutes
- MeOH:: methanol
- MIC:: minimum inhibitory concentration
- MS:: mass spectrometry
- NBS:: *N*-bromosuccinimide
- NMR:: nuclear magnetic resonance spectroscopy
- Ns:: nosyl, nitrobenzenesulfonyl
- Ppm:: parts per million
- q:: quadruplet
- rt:: room temperature
- s:: singlet
- t:: triplet
- TEA:: triethylamine
- THF:: tetrahydrofuran
- TFA:: trifluoroacetic acid
- TLC:: thin layer chromatography

The compounds of the present invention of formula (I), (I*), (A), (A*), (B) or (B*) can be prepared respectively by the following reaction schemes 1 to 7 depending on R¹.

It should be understood that the processes of schemes 1 to 7 can be adapted for preparing further compounds according to the invention. Further processes for the preparation of compounds according to the invention can be derived from the processes of schemes 1 to 7.

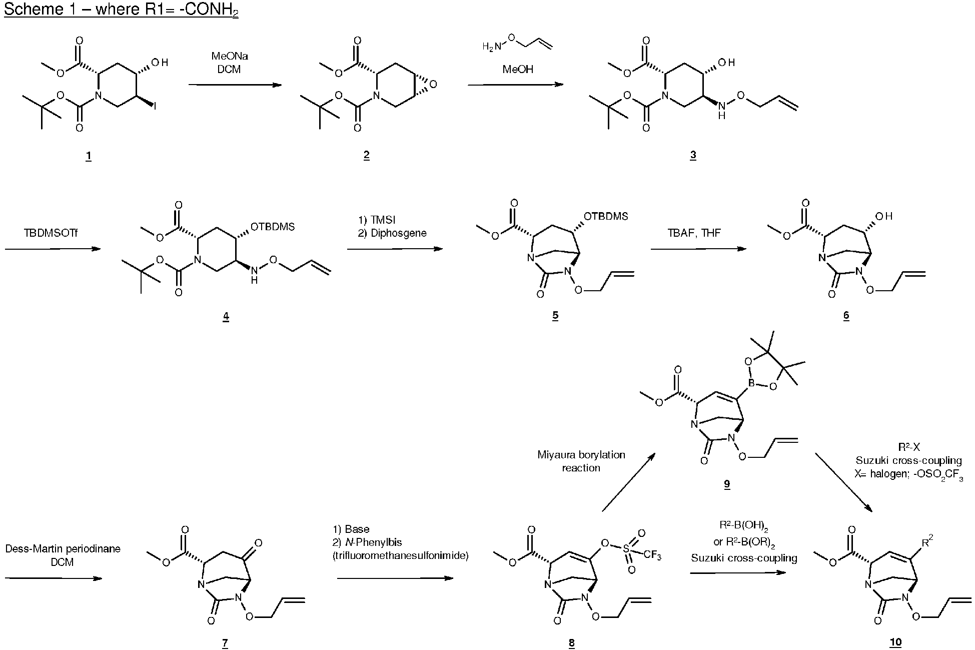

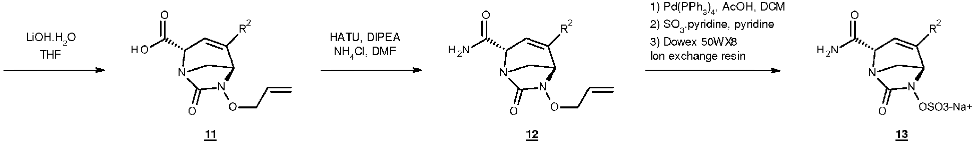

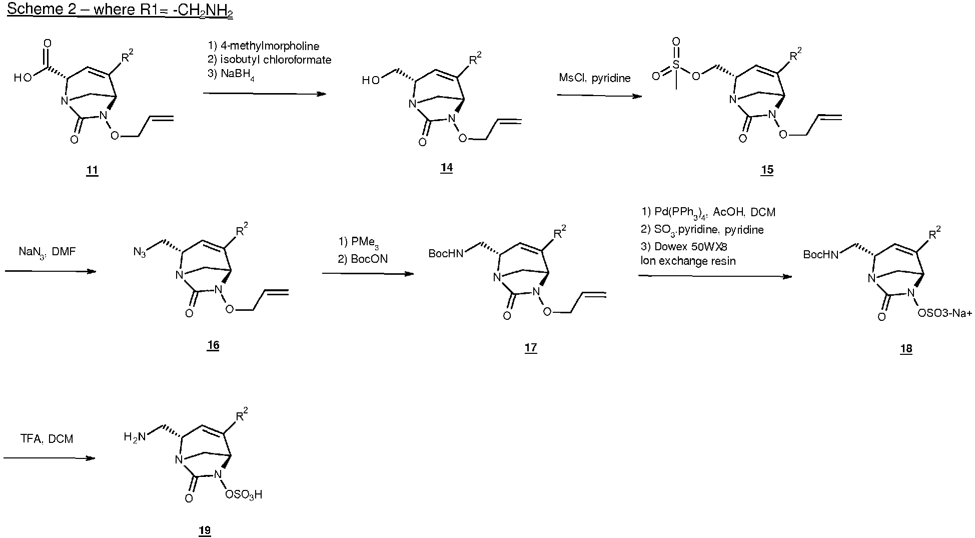

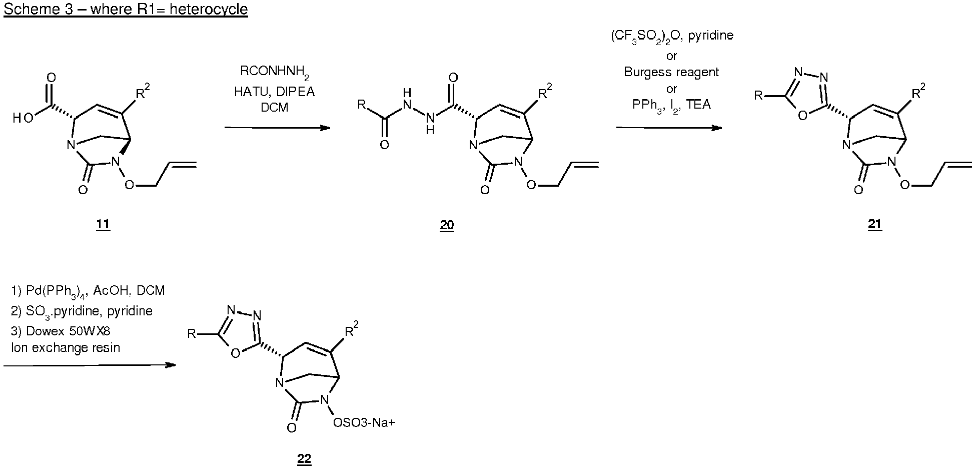

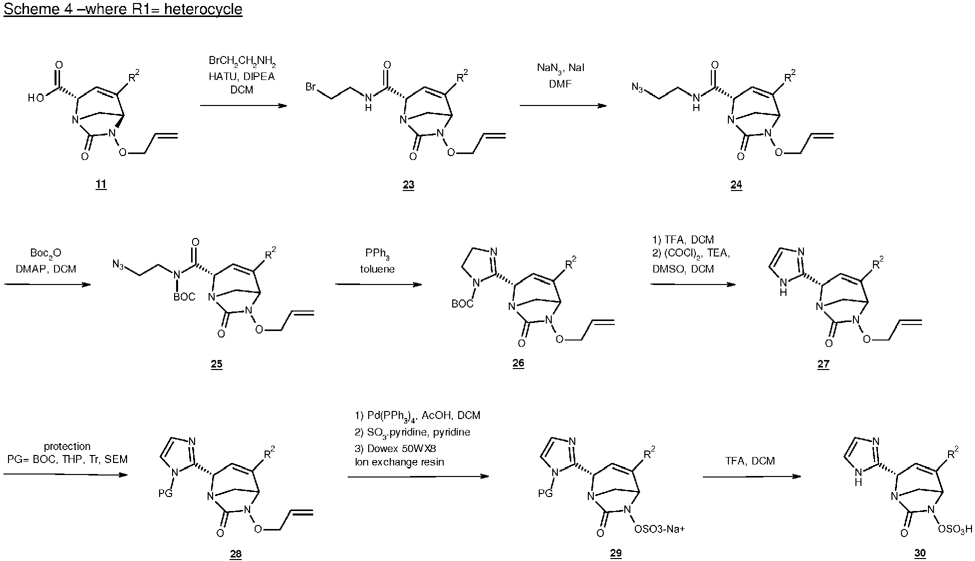

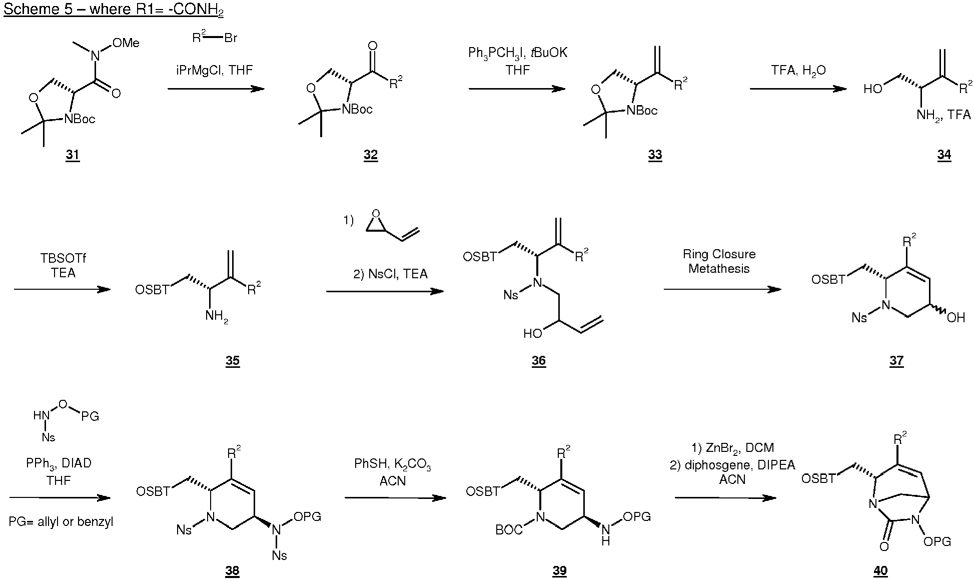

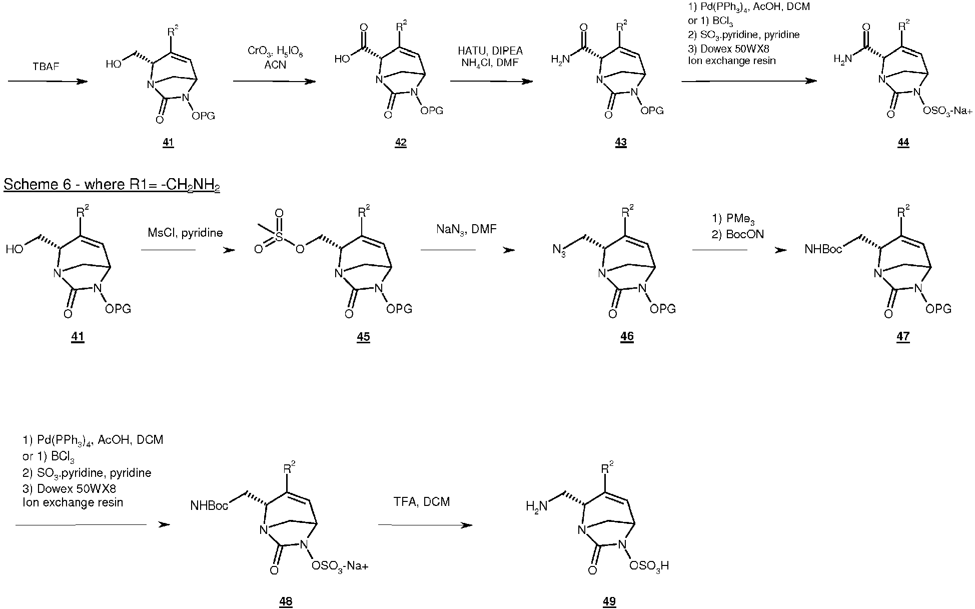

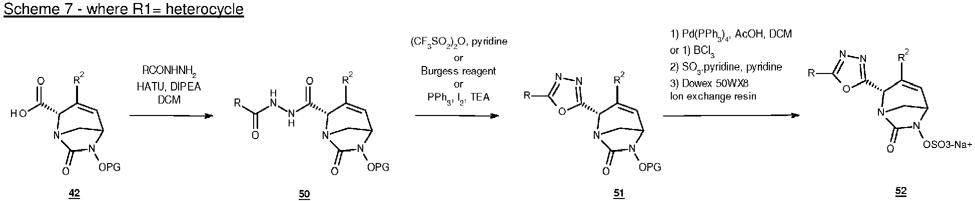

## Claims

1. A compound of formula (I) 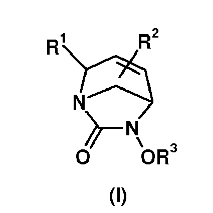 wherein
R¹ represents a carbon-linked 4-, 5- or 6-membered heterocycle saturated, partially or totally unsaturated or aromatic comprising at least one nitrogen atom, -CN, -(CH₂)ₘ-OQ¹, -(CH₂)m-OC(O)Q¹, -C(O)OQ¹, -(CH₂)ₘ-OC(O)OQ¹, -(CH₂)ₘ-OC(O)NQ¹Q², -C(O)NHQ¹, -C(O)NHOQ¹, -(CH₂)ₘ-NHS(O)₂NQ¹Q², -C(O)NH-NHQ¹, -C(O)O-NHQ¹, -(CH₂)ₘ-NHC(O)Q¹, - (CH₂)ₘ-NHS(O)₂Q¹, -(CH₂)ₘ-NHC(O)OQ¹, -(CH₂)ₘ-NHC(O)NQ¹Q², -(CH₂)ₘ-NHQ³, -(CH₂)ₘ-NH-C(NHQ³)=NQ⁴, (CH₂)ₘ-NH-CH=NQ³, -C(NHQ³)=NQ⁴, wherein the heterocycle is optionally substituted by one or more T¹;
R² represents a 4- to 10-membered heterocycle saturated, partially or totally unsaturated or aromatic optionally substituted by one or more T²;
R³ represents -SO₃H, -CFHCOOH or -CF₂COOH;
T¹, identical or different independently represents a fluorine atom, a (C₁-C₃)-alkyl, a (C₁-C₃)-fluoroalkyl, O-(C₁-C₃)fluoroalkyl, -(CH₂)ₙ-heterocycle wherein the heterocycle is a 4-, 5- or 6-membered heterocycle saturated, partially or totally unsaturated or aromatic comprising at least one nitrogen atom, -(CH₂)ₙOQ¹, -(CH₂)ₙ-C(O)ONHQ¹, -(CH₂)ₙ-CN, -(CH₂)ₙ-OC(O)Q¹, -(CH₂)ₙ-C(O)OQ¹, -(CH₂)ₙ-NHS(O)₂NQ¹Q², -(CH₂)ₙ-OC(O)OQ¹, -(CH₂)ₙ-OC(O)NHQ¹, -(CH₂)ₙ-C(O)NHQ¹, -(CH₂)ₙ-C(O)NHOQ¹, -(CH₂)ₙ-C(O)NH-NHQ¹, -(CH₂)ₙ-NHC(O)Q¹, -(CH₂)ₙ-NHS(O)₂Q¹, -(CH₂)ₙ-NHC(O)OQ¹, -(CH₂)ₙ-NHC(O)NQ¹Q², -(CH₂)ₙ-NHQ¹, -(CH₂)ₙ-NH-C(NHQ³)=NQ⁴, -(CH₂)ₙ-NH-CH=NQ³, (CH₂)ₙ-C(NHQ³)=NQ⁴, wherein the alkyl, fluoroalkyl, O-fluoroalkyl, and -(CH₂)ₙ-heterocycle are independently optionally substituted by one or more T³;
Q¹ and Q², identical or different independently represent a hydrogen atom, (C₁-C₃)alkyl, -(CH₂)_{q}-NHQ³, -(CH₂)_{q}-NH-C(NHQ³)=NQ⁴, (CH₂)_{q}-NH-CH=NQ³, (CH₂)ᵣ-C(NHQ³)=NQ⁴, -(CH₂)_{q}-OQ³, -(CH₂)ᵣ-CONHQ³, -(CH₂)ₙ-heterocycle, wherein the heterocycle is a 4-, 5- or 6-membered heterocycle saturated, partially or totally unsaturated or aromatic comprising at least one nitrogen atom; wherein the alkyl and -(CH₂)ₙ-heterocycle are independently optionally substituted by one or more T³; or
Q¹ and Q² and the nitrogen atom to which they are bonded form together a saturated or partially unsaturated 4-, 5- or 6-membered heterocycle optionally substituted by one or more T³;
Q³ and Q⁴, identical or different, independently represent a hydrogen atom or (C₁-C₃)alkyl;
T², identical or different, independently represents a fluorine atom, (C₁-C₃)alkyl, (C₁-C₃)fluoroalkyl, O-(C₁-C₃)fluoroalkyl, (X)ₚ-(CH₂)ₙ-(C₃-C₆)cycloalkyl, (X)ₚ-(CH₂)ₙ-(C₃-C₆)cyclofluoroalkyl, -(X)ₚ-(CH₂)ₙ-heterocycle wherein the heterocycle is a 4-, 5- or 6-membered heterocycle, saturated, partially or totally unsaturated or aromatic, -(X)ₚ(CH₂)ₜOQ⁵, (X)ₚ-(CH₂)ᵤ-CN, -(X)ₚ-(CH₂)ₜ-OC(O)Q⁵, (X)ₚ-(CH₂)ᵤ-C(O)OQ⁵, (X)ₚ-(CH₂)ₜ-OC(O)OQ⁵, (X)ₚ-(CH₂)ₜ-OC(O)NQ⁵Q⁶, (X)ₚ-(CH₂)ᵤ-C(O)NQ⁵Q⁶, (X)ₚ-(CH₂)ᵤ-C(O)ONQ⁵Q⁶ , (X)ₚ-(CH₂)ᵤ-C(O)NQ⁵OQ⁶, (X)ₚ-(CH₂)ᵤ-C(O)NQ⁵-NQ⁵Q⁶, (X)ₚ-(CH₂)ₜ-NQ⁵C(O)Q⁶, (X)ₚ-(CH₂)ₜ-NQ⁵S(O)₂NQ⁵Q⁶, (X)ₚ-(CH₂)ₜ-NQ⁵S(O)₂Q⁶, (X)ₚ-(CH₂)ₜ-NQ⁵C(O)OQ⁶, (X)ₚ-(CH₂)ₜ-NQ⁵C(O)NQ⁵Q⁶, (X)ₚ-(CH₂)ₜ-NQ⁵Q⁶, (X)ₚ-(CH₂)ₜ-NH-C(NHQ³)=NQ⁴, (X)ₚ-(CH₂)ₜ-NH-CH=NQ³, (X)ₚ-(CH₂)ᵤ-C(NHQ³)=NQ⁴, wherein the alkyl, fluoroalkyl, O-fluoroalkyl, (X)ₚ-(CH₂)ₙ-cycloalkyl, (X)ₚ-(CH₂)ₙ-cyclofluoroalkyl, -(X)ₚ-(CH₂)ₙ-heterocycle are independently optionally substituted by one or more T³;
Q⁵ and Q⁶, identical or different independently represent a hydrogen atom, (C₁-C₃)alkyl, -(CH₂)_{q}-NHQ³, -(CH₂)_{q}-NH-C(NHQ³)=NQ⁴, (CH₂)_{q}-NH-CH=NQ³, (CH₂)ᵣ-C(NHQ³)=NQ⁴, -(CH₂)_{q}-OQ³, -(CH₂)ᵣ-CONHQ³ -(CH₂)ₙ-heterocycle, wherein the heterocycle is a 4-, 5- or 6-membered heterocycle saturated, partially or totally unsaturated or aromatic comprising at least one nitrogen atom; wherein the alkyl and -(CH₂)ₙ-heterocycle are independently optionally substituted by one or more T³; or
Q⁵ and Q⁶ and the nitrogen atom to which they are bonded form together a saturated or partially unsaturated 4- to 6-membered heterocycle optionally substituted by one or more T³;
T³, identical or different, independently represents -OH, -NH₂, -CONH₂;
m, identical or different, independently represents 1 or 2;
n, identical or different, independently represents 0,1, 2 or 3;
t, identical or different, independently represents 0, 1, 2 or 3;
u, identical or different, independently represents 0, 1, 2 or 3;
q, identical or different, independently represents 2 or 3;
r, identical or different, independently represents 1, 2 or 3;
p, identical or different, independently represents 0 or 1 and when p is 0 t, identical or different is 0, 1, 2 or 3 and u, identical or different, is 0, 1, 2 or 3 and when p is 1 t, identical or different, independently represents 2 or 3 and u, identical or different, represents 1, 2 or 3; X, identical or different, independently represents O, S, S(O), S(O)₂ or N(Q³);
wherein
• any carbon atom present within a group selected from alkyl ; cycloalkyl ; fluoroalkyl ; cyclofluoroalkyl ; heterocycle can be oxidized to form a C(O) group;
• any sulphur atom present within an heterocycle can be oxidized to form a S(O) group or a S(O)₂ group ;
• any nitrogen atom present within a group wherein it is trisubstituted (thus forming a tertiary amine) or within an heterocycle can be further quaternized by a methyl group;
or their pharmaceutically acceptable salts, their corresponding zwitterion, or their optical isomers, racemates, diastereoisomers, enantiomers or tautomers.

2. A compound according to claim 1, wherein :
- R¹ represents a carbon-linked 4-, 5- or 6-membered heterocycle saturated, partially or totally unsaturated or aromatic comprising at least one nitrogen atom, -CN, -(CH₂)ₘ-OQ¹, - (CH₂)ₘ-OC(O)Q¹, -C(O)OQ¹, -(CH₂)ₘ-OC(O)OQ¹, -(CH₂)ₘ-OC(O)NQ¹Q², -C(O)NHQ¹, - (CH₂)ₘ-NHS(O)₂NQ¹Q², -C(O)NHOQ¹, -C(O)NH-NHQ¹, -C(O)O-NHQ¹, -(CH₂)ₘ-NHC(O)Q¹, - (CH₂)ₘ-NHS(O)₂Q¹, -(CH₂)ₘ-NHC(O)OQ¹, -(CH₂)ₘ-NHC(O)NQ¹Q², wherein the heterocycle is optionally substituted by one or more T¹, wherein m, Q¹, Q² and T¹ are as defined in claim 1; or
- R¹ represents -(CH₂)ₘNHQ³, -(CH₂)ₘ-NH-C(NHQ³)=NQ⁴, (CH₂)ₘ-NH-CH=NQ³, - C(NHQ³)=NQ⁴, wherein m, Q³ and Q⁴ are as defined in claim 1.

3. A compound according to claim 1, wherein :
- R¹ represents a carbon-linked 4-, 5- or 6-membered heterocycle saturated, partially or totally unsaturated or aromatic comprising at least one nitrogen atom, CN, -C(O)NHQ¹, - C(O)NHOQ¹, -C(O)NH-NHQ¹ or -(CH₂)ₘOQ¹ wherein the heterocycle is optionally substituted by one or more T¹, wherein m, Q¹ and T¹ are as defined in claim 1; or.
- R¹ represents -(CH₂)ₘNHQ³, -(CH₂)ₘ-NH-C(NHQ³)=NQ⁴, wherein m, Q³ and Q⁴ are as defined in claim 1.

4. A compound according to claim 1, R¹ represents a carbon-linked 4- to 6-membered heterocycle saturated, partially or totally unsaturated or aromatic comprising at least one nitrogen atom, -(CH₂)mOC(O)Q¹, -C(O)OQ¹, -(CH₂)ₘ-NHS(O)₂NQ¹Q², -(CH₂)ₘ-OC(O)OQ¹, - C(O)NHOQ¹, -C(O)NH-NHQ¹,-C(O)O-NHQ¹, -(CH₂)ₘ-NHC(O)Q¹, -(CH₂)ₘNHS(O)₂Q¹, - (CH₂)ₘ-NHC(O)OQ¹, -(CH₂)ₘ-NHC(O)NQ¹Q², -(CH₂)ₘ-NH-C(NHQ³)=NQ⁴, (CH₂)ₘ-NH-CH=NQ³ or -C(NHQ³)=NQ⁴, wherein the heterocycle is optionally substituted by one or more T¹, wherein m, Q¹, Q², Q³, Q⁴ and T¹ are as defined in claim 1.

5. A compound according to claim 1, wherein :
- R¹ represents -(CH₂)ₘ-NH-C(NHQ³)=NQ⁴, (CH₂)ₘ-NH-CH=NQ³, (CH₂)-C(NHQ³)=NQ⁴, wherein m, Q³ and Q⁴ are as defined in claim 1; or
- R¹ represents a carbon-linked 4-, 5- or 6-membered heterocycle saturated, partially or totally unsaturated or aromatic comprising at least one nitrogen atom, -(CH₂)ₘOC(O)Q¹, - C(O)OQ¹, -(CH₂)ₘ-OC(O)OQ¹, -(CH₂)ₘ-NHS(O)₂NQ¹Q², -C(O)NHOQ¹, -C(O)NH-NHQ¹, - C(O)O-NHQ¹, -(CH₂)ₘ-NHC(O)Q¹, -(CH₂)ₘNHS(O)₂Q¹, -(CH₂)ₘ-NHC(O)OQ¹, -(CH₂)ₘ-NHC(O)NQ¹Q² wherein the heterocycle is optionally substituted by one or more T¹ wherein m, Q¹, Q² and T¹ are as defined in claim 1.

6. A compound according to claim 1, wherein :
- R¹ represents -C(NHQ³)=NQ⁴, wherein m, Q³ and Q⁴ are as defined in claim 1; or
- R¹ represents a carbon-linked 4-, 5- or 6-membered heterocycle saturated, partially or totally unsaturated or aromatic comprising at least one nitrogen atom, -C(O)NHOQ¹ or - C(O)NH-NHQ¹, wherein the heterocycle is optionally substituted by one or more T¹, wherein m, Q¹ and T¹ are as defined in claim 1.

7. A compound according to claim 1, wherein R¹ represents a carbon-linked 4-, 5- or 6-membered heterocycle saturated, partially or totally unsaturated or aromatic comprising at least one nitrogen atom, optionally substituted by one or more T¹; -C(O)NHQ¹, -(CH₂)ₘ-NH-C(NHQ³)=NQ⁴ or -(CH₂)ₘNHQ³, wherein T¹, Q¹, Q³, Q⁴ and m are as defined in claim 1.

8. A compound according to anyone of claims 1 to 7, wherein R² represents a 4-, 5 or 6-membered heterocycle, saturated, partially or totally unsaturated or aromatic, comprising at least one nitrogen atom and optionally at least another heteroatom chosen among O, S, S(O), S(O)₂ or N, the heterocycle being optionally substituted by one or more T² as defined in claim 1.

9. A compound according to anyone of claims 1 to 8 of formula (I*) 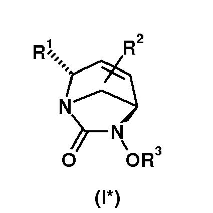 wherein R¹, R² and R³ are as defined in claims 1 to 8.

10. A pharmaceutical composition comprising at least one compound according to anyone of claims 1 to 9 with a pharmaceutically acceptable excipient.

11. A pharmaceutical composition according to claim 10, further comprising at least one or more antibacterial agent(s), preferably chosen among the following families: aminoglycosides, beta-lactams, glycylcyclines, tetracyclines, quinolones, fluoroquinolones, glycopeptides, lipopeptides, macrolides, ketolides, lincosamides, streptogramins, oxazolidinones and polymyxins alone or in mixture, preferably at least one of the antibacterial agent is a beta-lactam.

12. A pharmaceutical composition according to claim 11, wherein the further antibacterial agent is selected among the beta-lactam families, and more preferably among penicillin, cephalosporins, penems, carbapenems and monobactam, alone or in mixture.

13. A kit comprising:
- a pharmaceutical composition according to claim 10, and
- at least another composition comprising one or more antibacterial agent(s), preferably at least one of the antibacterial agent is a beta-lactam

14. A compound according to anyone of claims 1 to 9 or the composition according to claims 10 to 12, for use as a medicine.

15. A compound according to anyone of claims 1 to 9 for use as antibacterian agent and/or inhibitor of beta-lactamase.

16. A compound according to anyone of claims 1 to 9 or a composition according to claims 10 to 12, for use for the treatment or prevention of a bacterial infections, such as bacterial infection caused by bacteria producing one ore more beta-lactamase(s), preferably wherein the bacteria are chosen amongst gram-positive bacteria or gram-negative bacteria, preferably gram-negative bacteria.

17. A kit according to claim 13, for a simultaneous, separated or sequential administration to a patient in need thereof for the treatment or prevention of bacterial infections.
